(19) Europäisches Patentamt

European Patent Office

Office européen des brevets

(11) **EP 2 626 694 B1**

(12) **EUROPEAN PATENT SPECIFICATION**

(45) Date of publication and mention
of the grant of the patent:
**08.05.2019 Bulletin 2019/19**

(51) Int Cl.:
*G01N 27/407* (2006.01)     *G01N 33/00* (2006.01)

(21) Application number: **13154457.9**

(22) Date of filing: **07.02.2013**

(54) **Exhaust gas sensor module**

Abgassensormodul

Module de capteur de gaz d'échappement

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.02.2012 US 201261597574 P**

(43) Date of publication of application:
**14.08.2013 Bulletin 2013/33**

(73) Proprietor: **Cummins IP, Inc.
Minneapolis, MN 55432 (US)**

(72) Inventors:
• **Mitchell, Douglas A
Indianapolis, IN 46237 (US)**
• **Clerc, James C
Columbus, IN 47203 (US)**

• **Liu, Z. Gerald
Madison, WI 53717 (US)**
• **Manekar, Abhishek
Columbus, IN 47201 (US)**
• **Simonton, William L
Columbus, IN 47201 (US)**
• **Szailer, Tamas
Seymour, IN 47274 (US)**

(74) Representative: **Hoffmann Eitle
Patent- und Rechtsanwälte PartmbB
Arabellastraße 30
81925 München (DE)**

(56) References cited:
**EP-A1- 0 978 721     DE-A1- 10 203 310
DE-A1- 10 348 175**

EP 2 626 694 B1

## Description

## FIELD

This disclosure relates to sensing characteristics of a fluid, and more particularly to a fluid sensor module for sensing characteristics of a flowing fluid.

## BACKGROUND

Exhaust emissions regulations for internal combustion engines have become more stringent over recent years. For example, the regulated emissions of $NO_x$ and particulates from diesel-powered internal combustion engines are low enough that, in many cases, the emissions levels cannot be met with improved combustion technologies. Therefore, the use of exhaust after-treatment systems on engines to reduce harmful exhaust emissions is increasing. Typical exhaust after-treatment systems include any of various components configured to reduce the level of harmful exhaust emissions present in the exhaust gas. For example, some exhaust after-treatment systems for diesel-powered internal combustion engines include various components, such as a diesel oxidation catalyst (DOC), a particulate matter filter or diesel particulate filter (DPF), and a selective catalytic reduction (SCR) catalyst. In some exhaust after-treatment systems, exhaust gas first passes through the diesel oxidation catalyst, then passes through the diesel particulate filter, and subsequently passes through the SCR catalyst.

Each of the DOC, DPF, and SCR catalyst components is configured to perform a particular exhaust emissions treatment operation on the exhaust gas passing through or over the components. The DOC, DPF, and SCR catalyst each include a catalyst bed or substrate that facilitates the corresponding exhaust emissions treatment operation. Generally, the catalyst bed of the DOC reduces the amount of carbon monoxide and hydrocarbons present in the exhaust gas via oxidation techniques. The substrate of the DPF filters harmful diesel particulate matter and soot present in the exhaust gas. Finally, the catalyst bed of the SCR catalyst reduces the amount of nitrogen oxides ($NO_x$) present in the exhaust gas.

Generally, the catalyst bed of the SCR catalyst is configured to convert $NO_x$ (NO and $NO_2$ in some fraction) to $N_2$ and other compounds. SCR systems utilize a reductant (e.g., diesel exhaust fluid (DEF)) and the SCR catalyst to convert the $NO_x$. In most conventional SCR systems, ammonia is used to reduce $NO_x$. However, due to the undesirability of handling pure ammonia, most systems utilize an alternate compound such as urea, which vaporizes and decomposes to ammonia before entering the SCR catalyst. When just the proper amount and distribution of ammonia is available at the SCR catalyst under the proper conditions, the ammonia reduces $NO_x$ in the presence of the SCR catalyst. Currently available SCR systems can produce high $NO_x$ conversion rates allowing the combustion technologies to focus on power and efficiency. However, currently available SCR systems also suffer from several drawbacks. For example, one known drawback is the inability to effectively provide feedback control of the engine system based on the sensed characteristics of exhaust gas flowing through the SCR system.

Conventional methods for controlling operation of an engine and a reductant doser in an SCR system are based on an open-loop control system. Inputs to the open-loop control system include sensed characteristics of exhaust gas flowing through the system. One or more of the sensed characteristics are compared to a predetermined operating map to obtain an appropriate reductant dosing rate. Typically, the characteristics are sensed at a location upstream of the SCR catalyst of the SCR system. Often, to detect failures or accommodate correction of the map-generated reductant dosing rate, additional characteristics of the exhaust gas sensed at a location downstream of the SCR catalyst can be used. Although a control system employing sensors upstream and downstream of an SCR catalyst provides some benefits, the efficiency and accuracy of the system often suffers with such an arrangement.

Additionally, the design of sensors used in conventional exhaust after-treatment systems for sensing exhaust characteristics often promotes several drawbacks. Typical sensors used in exhaust after-treatment systems are point-measurement devices that sense the concentration of components of the exhaust gas at a single localized point within the exhaust stream. A controller then assigns a component concentration for all the exhaust gas flowing through the system based on the sensed concentration at the localized point. Often, the localized point is at an outer periphery or a center of the exhaust gas stream. In most systems, however, component concentrations within the exhaust gas stream can be poorly spatially distributed. Such poor spatial distribution of components within the exhaust gas can be caused by inadequate mixing of the reductant upstream of the SCR catalyst. Inadequate mixing of reductant upstream of the SCR catalyst can also result in poor distribution of $NO_x$ downstream of the SCR catalyst. Component concentration calculations for the entire exhaust gas stream based on readings taken from mal-distributed exhaust glow by point-measurement sensors upstream and downstream of the SCR catalyst may be inaccurate. Inaccurate component concentration calculations may lead to measurement errors and potentially negative effects on the efficiency and longevity of an exhaust after-treatment system, particularly an SCR system.

Further, certain probe-type sensors demand a certain exhaust gas flow rate through the probe and past the sensing device for accurate readings. Often, maintaining an adequate exhaust gas flow rate through the probe under a wide range of operating conditions is difficult.

DE-10203310-A1 discloses a device for measuring the gaseous components of a flowing gas mixture, comprising a gas feed, at least one sensor in contact with the gas mixture, and a mixing unit inserted into the flow of the gas mixture to homogenize the gas mixture before it reaches the sensor. The mixing unit is homogenized by 1-100%, preferably 20% of the gas mixture flowing in the gas feed. The mixing unit is arranged on the whole diameter of the gas feed or from the inner wall region up to the region of the central axis at a distance or along the whole inner periphery of the gas feed. The mixing unit is heated by a heating device.

## SUMMARY

The subject matter of the present application has been developed in response to the present state of the art, and in particular, in response to the problems and needs in the fluid sensing art that have not yet been fully solved by currently available sensors and sensing systems. Accordingly, the subject matter of the present application has been developed to provide a fluid sensor module and associated apparatus, systems, and methods for sensing component concentrations in a fluid stream that overcomes at least some shortcomings of the prior art approaches.

The present invention provides a sensor module for sensing characteristics of a fluid flowing through a fluid conduit and a method for sensing characteristics of a fluid flowing through a fluid conduit using the sensor module, as defined by the claims appended hereto.

The described features, structures, advantages, and/or characteristics of the subject matter of the present disclosure may be combined in any suitable manner in one or more embodiments and/or implementations. In the following description, numerous specific details are provided to impart a thorough understanding of embodiments of the subject matter of the present disclosure. One skilled in the relevant art will recognize that the subject matter of the present disclosure may be practiced without one or more of the specific features, details, components, materials, and/or methods of a particular embodiment or implementation. In other instances, additional features and advantages may be recognized in certain embodiments and/or implementations that may not be present in all embodiments or implementations. Further, in some instances, well-known structures, materials, or operations are not shown or described in detail to avoid obscuring aspects of the subject matter of the present disclosure. The features and advantages of the subject matter of the present disclosure will become more fully apparent from the following description and appended claims, or may be learned by the practice of the subject matter as set forth hereinafter.

## BRIEF DESCRIPTION OF THE DRAWINGS

In order that the advantages of the subject matter may be more readily understood, a more particular description of the subject matter briefly described above will be rendered by reference to specific embodiments that are illustrated in the appended drawings. Understanding that these drawings depict only typical embodiments of the subject matter and are not therefore to be considered to be limiting of its scope, the subject matter will be described and explained with additional specificity and detail through the use of the drawings, in which:

FIG. 1 is a schematic block diagram of an internal combustion engine system having an engine, exhaust gas after-treatment system, a sensor module, an optional reductant delivery system, and an engine control unit, in accordance with a representative embodiment;

FIG. 2 is a cross-sectional side view of an SCR system having one or more fluid sensor modules installed therein, in accordance with yet another representative embodiment;

FIG. 3 is a front perspective view of sample probe for a sensor module, in accordance with another representative embodiment;

FIG. 4 is a front view of the sample probe of FIG. 2;

FIG. 5 is a schematic illustration of an equal area sampling methodology;

FIG. 6 is a rear view of the sample probe of FIG. 2;

FIG. 7 is a side view of the sample probe of FIG. 2;

FIG. 8 is a cross-sectional front view of the sample probe of FIG. 2, as viewed from section line A-A of FIG. 6;

FIG. 9 is a cross-sectional front view of a sample probe and sensor installed together within a fluid conduit to form a sensor module, in accordance with another representative embodiment;

FIG. 10 is a schematic, cross-sectional side view of the sensor module of FIG. 8, as viewed from section line B-B;

FIG. 11 is a schematic, cross-sectional top view of the sensor module of FIG. 8, as viewed from section line C-C; and

FIG. 12 is a cross-sectional side view of an SCR system having a bypass version of the fluid sensor module installed therein, in accordance with yet another representative embodiment.

**DETAILED DESCRIPTION**

Reference throughout this specification to "one embodiment," "an embodiment," or similar language means that a particular feature, structure, or characteristic described in connection with the embodiment is included in at least one embodiment of the present disclosure. Appearances of the phrases "in one embodiment," "in an embodiment," and similar language throughout this specification may, but do not necessarily, all refer to the same embodiment. Similarly, the use of the term "implementation" means an implementation having a particular feature, structure, or characteristic described in connection with one or more embodiments of the present disclosure, however, absent an express correlation to indicate otherwise, an implementation may be associated with one or more embodiments.

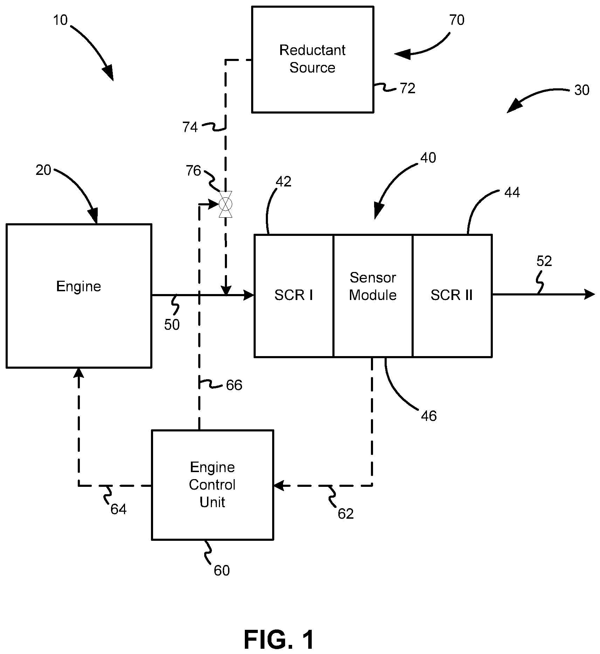

FIG. 1 depicts one embodiment of an internal combustion engine system 10. The main components of the engine system 10 include an internal combustion engine 20 and an exhaust gas after-treatment system 30 coupled to the engine. The internal combustion engine 20 can be a compression ignited internal combustion engine, such as a diesel-powered engine. Within the internal combustion engine 20, the air from the atmosphere is combined with fuel to power the engine. Combustion of the fuel and air produces exhaust gas. At least a portion of the exhaust gas generated by the internal combustion engine 20 is operatively vented to the exhaust gas after-treatment system 30 as indicated by directional arrow 50.

Generally, the exhaust gas after-treatment system 30 is configured to remove various chemical compound and particulate emissions present in the exhaust gas received from the engine 20. The exhaust gas after-treatment system 30 can include any of various exhaust treatment devices, such as diesel oxidation catalysts, diesel particulate filters, and SCR systems. Although the exhaust gas after-treatment system 30 may include one or more other devices or systems, in the illustrated embodiment, only an SCR system 40 is shown. In exhaust flow direction 50, exhaust flows from the engine 20 and through the SCR system 40 before exiting the SCR system as indicated by directional arrow 52.

The SCR system 40 includes an SCR catalyst device with a first SCR catalyst bed, substrate, or brick 42 upstream of a second SCR catalyst bed, substrate, or brick 44. In other words, the SCR system 40 includes an upstream SCR catalyst bed 42 and a downstream SCR catalyst bed 44.

The SCR system 40 further includes a sensor module 46 positioned between the upstream and downstream SCR catalyst beds 42, 44. The sensor module 46 receives exhaust gas from the upstream SCR catalyst bed 42 and senses a component (e.g., species) concentration in the exhaust gas before the exhaust gas flows into the downstream catalyst bed 44. In certain implementations, the SCR system 40 includes a single housing that houses the upstream SCR catalyst bed 42, downstream SCR catalyst bed 44, and sensor module 46. The component concentration reading or measurement is communicated over a communication line 62 to an engine control unit 60 that calculates a component concentration of the entire exhaust gas flow using an averaging technique based on the component concentration reading. In certain implementations, the engine control unit 60 is a separate exhaust after-treatment or SCR system control module which is electrically coupled to an engine control module.

The exhaust gas after-treatment system 30 can also include a reductant delivery system 70 which is located upstream of the upstream SCR catalyst bed 42. The reductant delivery system 70 includes a reductant source 72, a flow line connecting the reductant source to the exhaust gas stream as represented by directional arrow 50, and a control valve 76 which is operable to inject a dose of the reductant into the exhaust gas prior to the gas entering the SCR catalyst beds 42, 44. The injected reductant (or broken-down byproducts of the reductant, such as when urea is reduced to form ammonia) reacts with $NO_x$ in the presence of the SCR catalyst to reduce $NO_x$ in the exhaust gas to less harmful emissions, such as $N_2$ and $H_2O$. The SCR catalyst beds 42, 44 can be any of various catalysts known in the art. For example, in some implementations, the SCR catalyst beds 42, 44 each is a vanadium-based catalyst, and in other implementations, the SCR catalyst beds each is a zeolite-based catalyst, such as a Cu-Zeolite or a Fe-Zeolite catalyst.

In order to achieve a desired $NO_x$ reduction efficiency, fuel efficiency, and ammonia slip reduction, the calculated component concentration is used to control the operating conditions of the engine 20 via a communication line 64 and/or dosing of the reductant via a communication line 66 to the control valve 76 of the reductant delivery system 70. For example, in some implementations the calculated component concentration is used to generate a reductant dosing rate command from one or more predetermined maps. Alternatively, or additionally, the calculated component concentration can be used to detect failure of the SCR catalyst bed 42 and/or provide feedback correction of a dosing rate command. In such a mid-bed correction configuration, the sensor module 46 can effectively replace both a traditional sensor placed upstream of the SCR catalyst for reductant dosing rate determination purposes and a traditional sensor placed downstream of the SCR catalyst for on-board diagnostics (OBD) and correction purposes.

The spatial distribution of reductant and $NO_x$ at the inlet of the upstream SCR catalyst bed 42 affects the efficiency of the reduction of $NO_x$ by the upstream and downstream SCR catalyst beds 42, 44. Moreover, the distribution of reductant and $NO_x$ can be highly non-uniform and can vary significantly over an engine operating period. Non-uniformity of the spatial distribution of reductant and $NO_x$ at the inlet of the SCR catalyst bed 42 often translates into non-uniformity of the spatial distri-

bution of reductant and $NO_x$ at the outlet of the SCR catalyst bed 42 and at the sampling location of the sensor module 46. Such spatial distribution non-uniformity typically is exhibited throughout the SCR system 40, including at the inlet and outlet of the second SCR catalyst bed 44. Because of the possibility of non-uniform reductant and $NO_x$ spatial distribution between the first and second SCR catalyst beds 42, 44, the sensor module 46 is configured to capture a sample of the exhaust gas exiting the first SCR catalyst bed that more accurately represents the component characteristics of the entire exhaust gas compared to conventional probe-type sensors. In this manner, the sensor module 46 promotes accurate control of the reductant dosing system, efficient reduction of $NO_x$ by the SCR system 40, an increase in fuel efficiency, and a decrease in ammonia slip.

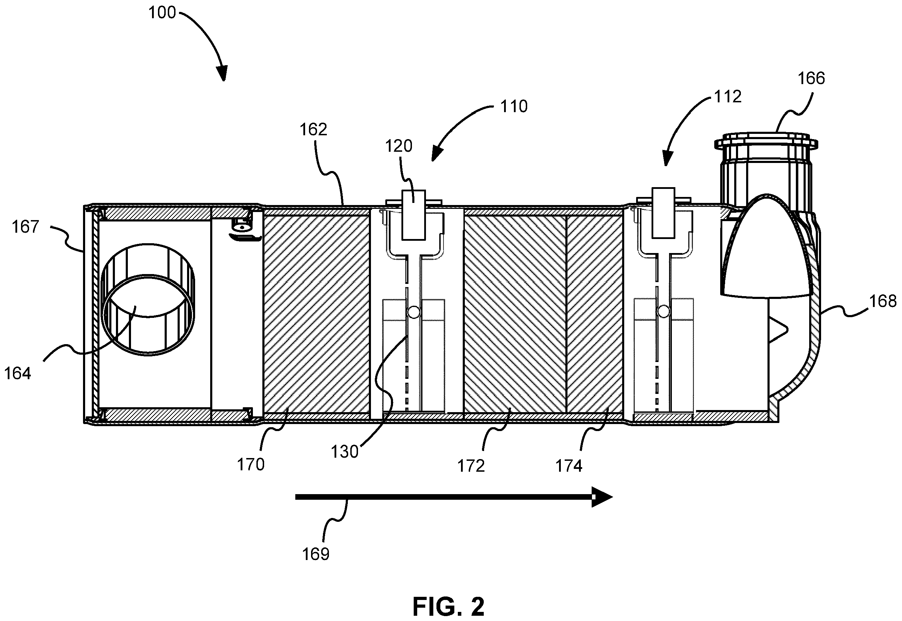

In accordance with another representative embodiment of the exhaust after-treatment system 100 illustrated in FIG. 2, a sensor module 110 similar to the sensor module 46 described above is utilized to sense the characteristics of exhaust flowing through the system. The exhaust after-treatment system 100 is an SCR system that includes a housing 162 within which an upstream SCR catalyst bed 170, a downstream SCR catalyst bed 172, and an ammonia oxidation (AMOX) catalyst bed 174 are housed. The housing 162 is substantially cylindrically shaped with an exhaust inlet 164 and an exhaust outlet 166. In operation, exhaust gas enters the housing through the inlet 164, flows from an upstream end 167 of the housing to a downstream end 168 in an exhaust gas flow direction 169, and exits the housing through the outlet 166. As the exhaust gas flows through the housing, the entirety of the exhaust gas stream flows through the upstream SCR catalyst bed 170, the downstream SCR catalyst bed 172, and the ammonia oxidation (AMOX) catalyst bed 174. Each of the catalyst beds 170, 172, 174 performs a specific emissions reduction operation on the exhaust gas as it passes through the beds.

The upstream and downstream catalyst beds 170, 172 define first and second portions or bricks (e.g., separate halves) of an SCR catalyst. Traditionally, the housing of an SCR system of an individual exhaust line houses a single SCR catalyst with a monolithic, one-piece catalyst bed. In the illustrated embodiment, however, the traditional SCR catalyst has been divided into two separate and spaced-apart portions (i.e., the upstream and downstream SCR catalyst beds 170, 172). The AMOX catalyst 174 in the illustrated embodiment is coupled to (e.g., integrated with) the downstream SCR catalyst bed 172. However, in certain other embodiments, the after-treatment system 100 does not have an AMOX catalyst, or has an AMOX catalyst that is separate from the downstream SCR catalyst bed 172. In one specific implementation as an example, the upstream and downstream SCR catalyst beds 170, 172 each have an approximately 13-inch diameter and 6-inch axial length. If present, the AMOX catalyst 174 can have an approximately 13-inch diameter and 3-inch axial length. Of course, in other implementations, the SCR catalyst beds 170, 172 and AMOX catalyst 174 can have any of various other sizes and shapes.

The space defined within the housing 162 between the upstream and downstream SCR catalyst beds 170, 172 is occupied by the sensor module 110, which includes both a sensor 120 and a sample probe 130. The sensor module 110 can be coupled to the housing 162 using any of various techniques. In the illustrated embodiment, the sample probe 130 of the sensor module 110 is secured to an interior surface of the housing 162 using a coupling technique, such as one or more of an adhering, a fastening, or a welding technique, while the sensor 120 can inserted through the housing 162 until a sensing tip of the sensor is correctly positioned with an appropriate sampling region.

Similar to the operation of sensor module 46 described above, sensor module 110 entrains a sample portion of the exhaust gas stream flowing from the outlet of the first SCR catalyst bed 170 and senses characteristics of the entrained gas before reintroducing the sample portion back into the exhaust gas stream. The sensed characteristics are reported to an engine control unit or reductant dosing controller (not shown) for precise and accurate control of the reductant dosing characteristics of the SCR system 100. In this manner, the sensor module 110 provides mid-bed reductant dosing control and/or correction capabilities without the need for an upstream static mixer and mixing tube to mix and evenly distribute the exhaust gas prior to sensing the exhaust gas.

Optionally, the SCR system 100 includes a second sensor module 112 similar to the first sensor module 110, but positioned downstream of the second SCR catalyst bed 172. For example, the SCR system 100 includes a second space defined within the housing 162 between the downstream SCR catalyst bed 172 and the downstream end 168 of the housing. The second sensor module 112 can be positioned within this second space, but alternatively may also be rotated ninety degrees and positioned in a suitable location within the SCR system's exhaust outlet 166 or within the exhaust piping that is further downstream from the exhaust outlet 166.

Like the first sensor module 110, the second sensor module 112 entrains a sample portion of the exhaust gas stream flowing from the outlet of the second SCR catalyst bed 172 or AMOX catalyst bed 174, and senses characteristics of the entrained portion. The sensed characteristics are reported to an engine control unit, or exhaust after-treatment controller, for controlling operation of the exhaust after-treatment system. In one embodiment, the first sensor module 110 is configured to measure a concentration of ammonia in the exhaust gas exiting the first SCR catalyst bed 170. More specifically, the sensor of the first sensor module 110 is an ammonia concentration sensor. The second sensor module 112 can be configured to measure a concentration of $NO_x$ in the exhaust gas exiting the second SCR catalyst bed 172. More specifically, the sensor of the second sensor module 112 is a NO$_x$ concentration sensor.

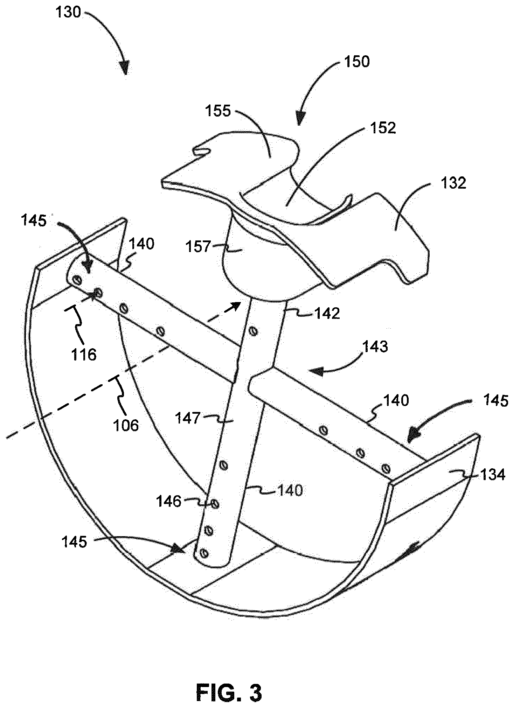

The sample probe 130 of the sensor module 110 is illustrated with more detail in FIG. 3. As can be seen, the sample probe 130 includes at least two sample arms 140, 142 which are coupled together at a central portion 143 and extend radially outward from the central portion to an outward end 145 which is coupled to either an upper fairing 132 or a lower fairing 134, which can be arcuate shaped. The upper fairing 132 and lower fairing 134 in turn are attached to the interior surface of the housing 160 of FIG. 2 which defines the fluid conduit into which the sample probe 130 is installed. In the illustrated embodiment, the upper and lower fairings 132, 134 are non-continuous, or separate and disconnected components.

Each sample arm is defined as either a single flow sample arm 140 or an aggregate flow sample arm 142. Generally, sample portions of the captured fluid entering the single flow sample arms 140 flow into and combine with sample portions entering and flowing through the aggregate flow sample arm 142. The sample probe 130 illustrated in FIG. 3 includes one aggregate flow sample arm 142 and three single flow sample arms 140, with the four arms being equally angularly-spaced apart from each other. However, in other embodiments, the sample probe can include fewer or more than four arms, with the arms being equally angularly-spaced apart from each other or with different angular intervals between the arms.

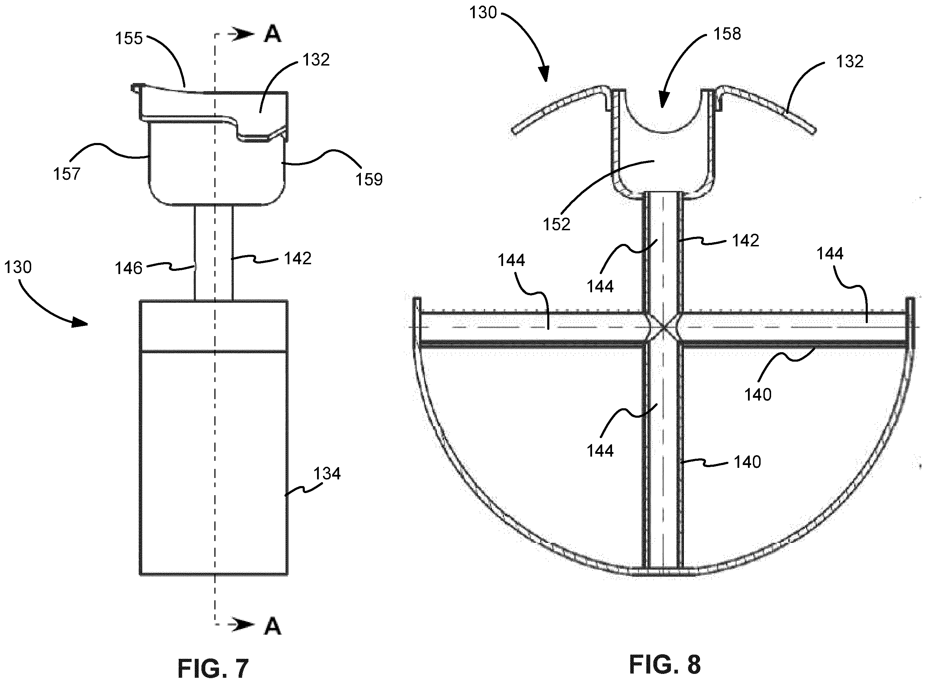

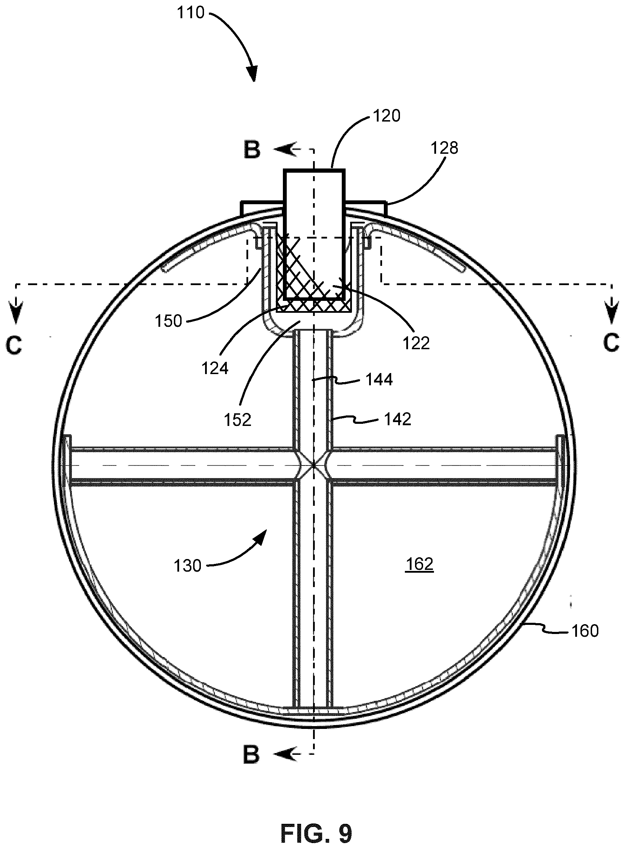

The sample arms 140, 142 are hollow and each include a fluid flow channel 144 formed therein (see FIGS. 8, 9). The fluid flow channel 144 of each sample arm can have any of various cross-sectional shapes, such as, for example, circular, elliptical, triangular, polygonal, and the like. The sample arms 140, 142 each include a set of inlet apertures 146 through which a sample portion 116 of a main fluid flow 106 is captured and directed into the fluid flow channels of the sample arms. As shown in FIG. 3, the inlet apertures 146 can be formed in respective upstream facing surfaces 147 of the sample arms 140, 142 such that each of the inlet apertures faces an upstream direction (i.e., normal to a fluid flow direction). In other words, the central axes of the inlet apertures 146 are substantially parallel to the fluid flow direction. In alternative embodiments, however, the inlet apertures 146 can also be angled into the side (or top and bottom) surfaces of the sample arms as well as in other non-normal locations/orientations as may be deemed appropriate. For example, in such alternative embodiments, the apertures 146 face directions angled with respect to the fluid flow direction. In other words, the central axes of the angled inlet apertures are substantially non-parallel to the fluid flow direction.

Further, the inlet apertures 146 of a respective sample arm 140, 142 are radially aligned along the respective arm from a location proximate a center portion of the sample arm to a location near or proximate the radially outward end 145 of the sample arm. In the illustrated embodiment, each single flow sample arm 140 includes four inlet apertures 146 while the aggregate flow sample arm 142 includes a single inlet aperture. However, in other embodiments, each sample arm may include fewer or more inlet apertures. As shown, the inlet apertures 146 are substantially circular-shaped. However, in other embodiment, the inlet apertures 146 can have other shapes, such as polygonal, elliptical, rectangular, triangular, and the like.

The sample probe 130 further includes a sensor well 150 that is located at the outward end 145 of the aggregate flow sample arm 142. The sensor well 150 is a blunt, rounded body that encloses an interior volume 152 in fluid communication with the fluid flow channel 144 formed into the aggregate flow sample arm 142. The sensor well 150 includes an outer portion 155, which opens to an interior surface of the fluid conduit or housing into which the sample probe is installed, with the upper fairing 132 providing a contact interface with the interior surface of the fluid conduit at both the upstream end and to either side of the sensor well to help secure the sample probe within the fluid conduit. Also shown in FIG. 3, the sensor well 150 includes a front portion 157 having a blunt, rounded surface for diverting the fluid flowing through the fluid conduit around the sensor well.

The sample probe 130 is installed within the fluid conduit or housing such that the conduit or housing extends completely around the sample probe 130 and causes the fluid flowing through the conduit to either pass between the sample arms 140, 142 as the main fluid flow 106, or into the inlet apertures 146 and through the interior passages of the sample probe 130 as the sample portion 116 of the fluid flow.

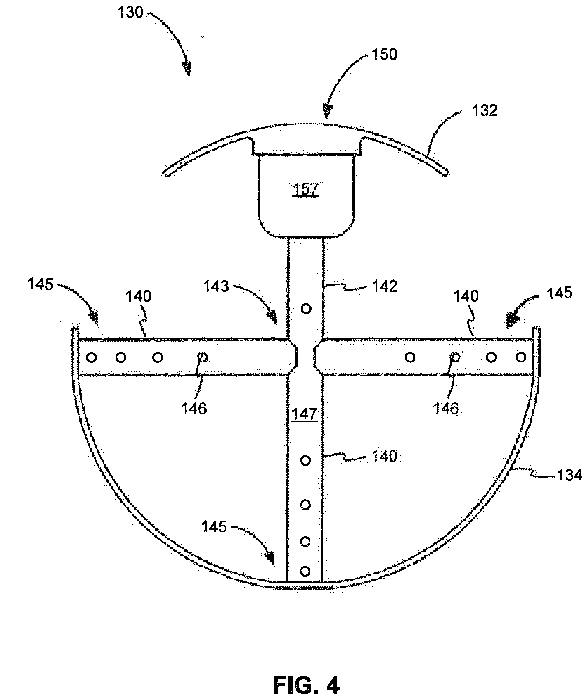

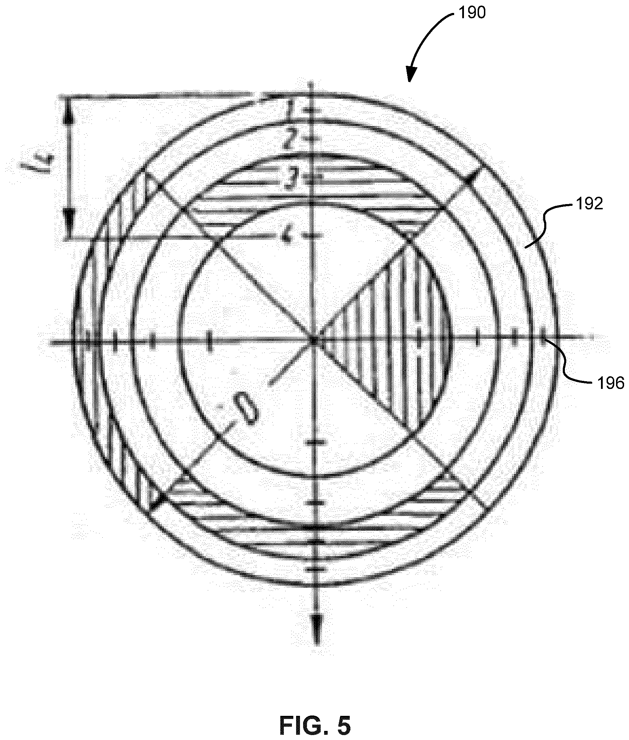

A front view of the sample probe 130 is provided in FIG. 4, which shows that the inlet apertures 146 of the single flow sample arms 140 and the aggregate flow sample arm 142 are equally sized with each other, but are spaced closer together near the outer ends 145 of the single flow sample arms 140. This is because the sample probe 130 is configured to take a sample of the main fluid flow having characteristics (e.g., species concentrations) that accurately represent the characteristics of the main fluid flow. Accordingly, the sample probe 130 accounts for the possibility of non-uniform fluid distribution patterns (i.e., non-uniform species mass fraction distribution) across a cross-section of a fluid stream flowing through a conduit by taking multiple samples of the fluid along multiple radially-spaced annular segments of the fluid stream, in accordance with the equal area sampling methodology 190 illustrated in FIG. 5.

As shown in the diagram of FIG. 5, one technique for equal area sampling 190 is facilitated by dividing the cross-section of the main fluid flow in the fluid conduit into N (e.g. 16) arc-shaped sections (e.g. arc sections) 192 of equal area. Equal area sampling is achieved by positioning each inlet aperture 146 at a location on the sample arm 140 which corresponds with a point 196 within a respective arc section 192. For example, the embodiment of FIGS. 4 and 5 uses a tangential method wherein the sample points within the arc sections are associated with a midpoint of the arc sections. In other embodiments using different methods, such as a log-linear or a log-chebyshev method, the points within the annular sections may be associated with a center of a pre-defined fluid velocity distribution within the annular sections.

The distance $l_j$ of the inlet apertures 146 from the interior surfaces of the fluid conduit may be scaled in accordance with a parametric relationship which depends upon the diameter "D" of the fluid conduit and the number "n" of inlet apertures 146 in each single flow sample arm 140. For precise calculations, the following equation can be used:

$$ l_j = \frac{D}{2}\left(1 - \sqrt{\frac{2n - 2j + 1}{2n}}\right) $$

For calculations requiring a lower level of resolution, the following relationship can be used, with $k_1$ to $k_4$ equal to 3.3, 10.5, 19.4 and 32.3, respectively, when the number of inlet apertures 146 in each sample arm is four.

$$ l_j = k_j\left(\frac{D}{100}\right) $$

Because the areas of the arc sections are the same, the radial width of each arc section decreases in the radially outward direction. Accordingly, the distance between adjacent apertures correspondingly decreases in the radially outward direction. As shown in FIG. 4, for example, sample probe 130 has three single flow sample arms 140, each with four variably-spaced and equally-sized inlet apertures 146. The distances between the inlet apertures 146 of the single flow sample arms 140 increase in a radially inward direction. In other words, the apertures 146 are closer to each other at the radially outward portion 145 of the arms 140 than the center portion 143 portion of the arms. The aggregate flow sample arm 142 may include only a single inlet aperture corresponding to a center arc section, with the remaining three sample points in the pattern being omitted to provide spacing for the sensor well 150 at the outward portion 145 of the aggregate flow sample arm 142. It has been found, nevertheless, that the illustrated configuration of the inlet apertures 146 in the sampling probe 130 is substantially accurate in capturing any non-uniform mass fraction distributions which may exist across the cross-section of the fluid stream passing through the fluid conduit. Furthermore, the size and number of inlet apertures 146 can be selected to produce a sampled volumetric flow rate which is 1% to 4% of the total volumetric flow rate flowing through the fluid conduit.

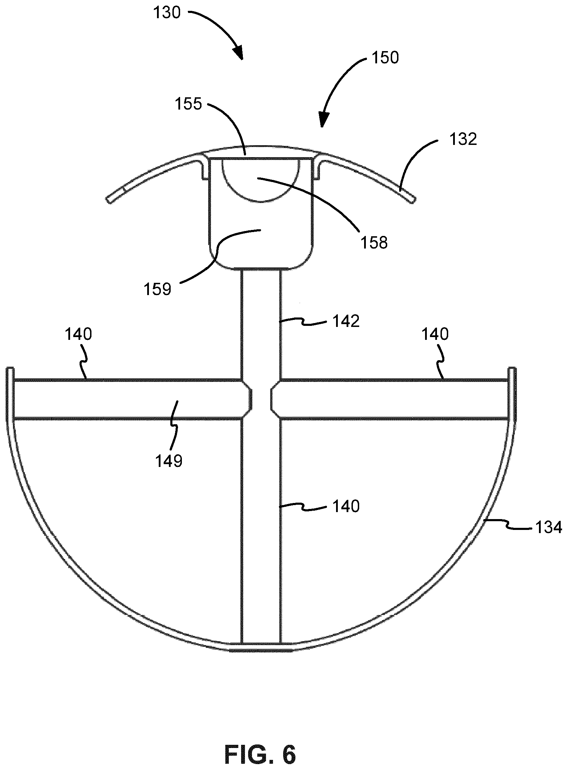

A rear view of the sample probe 130 showing the downstream surfaces 149 of the sample arms 140, 142 and the back portion 159 of the sensor well 150 is provided in FIG. 6. The back portion 159 includes a discharge aperture 158 which allows the captured sample portion of the flow to exit the interior volume of the sensor well and return to the main flow. As shown, the discharge aperture may have a substantially semi-circular cross-sectional shape, which facilitates a desired flow pattern of exhaust gas exiting the sensor well.

FIGS. 7 and 8 provide a side and cross-sectional front view of the isolated sample probe 130, respectively. As can be seen, each of the sample arms 140, 142 includes a fluid flow channel 144 which directs the captured sample portion of fluid towards the sensor well 150, where it then exits the sample probe 130 through the discharge aperture 158.

The cross-sectional front view of FIG. 8 is repeated in FIG. 9, but with the sample probe 130 being installed together with the sensor 120 within the fluid conduit or housing 160 of the SCR system to form the sensor module 110. The sensor 120 is installed through the wall of the fluid conduit 160 and positioned deep within the sensor well 150 so that a sensing tip 122 is located proximate the opening 148 between the aggregate flow sample arm 142 and the interior volume 152 of the sensor well 150.

The sensor 120 can be secured in place against the outside surface of the fluid conduit with a sensor receptacle 128. In the embodiment shown, the sensor 120 or sensor receptacle 128 can also include a condensing screen or basket 124 which may envelope the sensor 120 to protect the sensor from moisture and corrosive liquids. The spacing of the holes in the screen are small enough to prevent any moisture droplets from passing through the screen to contact the sensing tip 122 of the sensor 120, but still large enough to allow gases and vapors to come into contact with the sensing element.

Also shown in FIG. 9, the fluid flow channels 144 of each sample arm 140, 142 can have any of various hydraulic diameters. In some embodiments, the channels 144 have a hydraulic diameter between about 0.00635m (0.25 inches) and 0.0508m (2 inches) (e.g., between about 0.00635 m (0.25 inches) and 0.01905 m (0.75 inches) in some implementations). In one specific implementation, and as one example, the sample arms 140, 142 each define an approximately one-inch hydraulic diameter fluid flow channel 144 and the inlet apertures 146 formed in the sample arms each has an approximately 4.75 mm diameter. Because the size of the sample arms 140, 142 is relatively small compared to the size of the fluid channel 162 formed by the housing 160 of the SCR system, the sample probe 130 is minimally intrusive or obstructive (i.e., reduces the effect of the sensor module on the spatial distribution of fluid flow velocity and species).

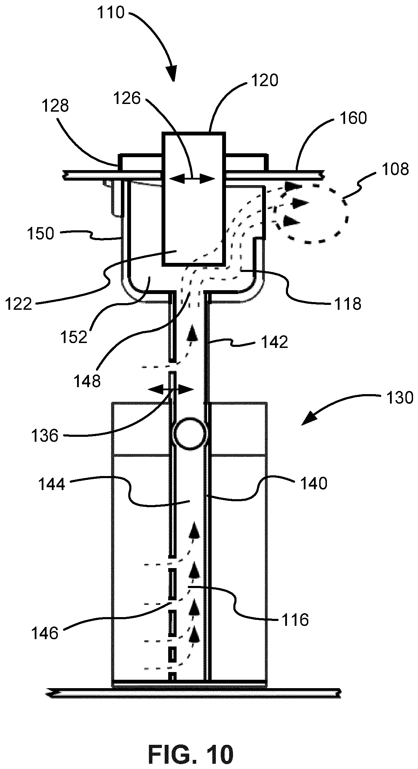

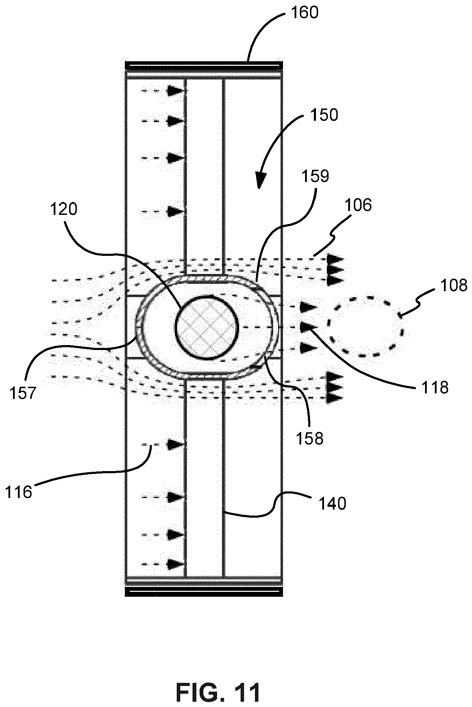

The flowpaths of the various fluid flows through sample probe 130 and around the sensing tip 122 of the sensor 120 are illustrated in FIGS. 10 and 11, which are cross-sectional views of the sample probe 130 and sensor well 150, respectively. Referring first to FIG. 10, the sample portions 116 of the main fluid flow passing through the fluid conduit 160 enter the sample probe 130 through the inlet apertures 146 formed in the sample arms 140, 142. The sample portions 116 pass into the fluid flow channels 144 inside the sample arms, where they are combined and directed towards sensor well 150, entering the internal volume 152 of the sensor well through opening 148 as an averaged combined sample flow 118 containing a representative mix of all the constituents of the exhaust gas currently passing around and between the sample arms of the sample probe 130. The combined sample flow 118 is then drawn across and around the sensing tip 122 of the sensor 120 which takes measurements of the combined sample flow 118 within the internal volume 152. The combined sample flow 118 then makes its way towards the discharge aperture 158 where it is drawn back out of sample probe 130 to recombine with the main fluid flow.

The axial position 136 of the sample arms 140, 142 relative to the axial position 126 of the sensor tip 122 can be adjusted so that most of the averaged combined sample flow 118 turns after entering the sensor well 150 through opening 148 and passes parallel to the bottom face of the sensor tip 122 rather than impacting directly against the bottom face. In making this adjustment, either the axial location 136 of the sample arms 140, 142 relative to the sensor well 150 or the axial location 126 of the sensor 120 relative to the sensor well 150 can be moved. In one aspect, for instance, the forward-facing surfaces of the sample arms 140, 142 can be made flush with the forward-facing surface of the sensor well 150, so that the averaged combined sample flow 118 enters the interior volume 152 through opening 148 at the 3 o'clock position near the front portion of the sensor well and exits at the 9 o'clock position through the discharge aperture in the back-facing surface of the sensor well.

A passive negative pressure inducing feature for enhancing the flow of the sample portions 116 through the sample probe 130 is illustrated in FIG. 11. For example, in addition to providing a protected internal volume 152 for sensing the combined sample flow 118, the sensor well 150 also provides a front portion 157 having a blunt rounded front surface which diverts and redirects the main fluid flow 106 passing around the sensor well. The sensor well 150 further provides a back portion 159 having the discharge aperture 158 formed therein, and which further can have a shape which encourages the main fluid flow to separate from the back portion 159 of the sensor well 150 when the main fluid flow 106 reaches a minimum speed. The separation of the main fluid flow 106 from the back portion 159 of the sensor well 150 causes a low pressure region 108 to form adjacent the sensor well and proximate the discharge aperture 158, which in turn operates to draw the combined sample flow 118 out from the internal volume 152 of the sensor well. This same low pressure region 108 can be seen proximate the discharge aperture 158 in FIG. 10.

Although the discharge aperture 158 is located in the rear surface of the back portion 159 and the low pressure region 108 is shown immediately behind the sensor well 150 in FIGS. 10 and 11, it is to be appreciated that other locations for the one or more discharge apertures and other shapes for the sensor well are also possible for creating the passive negative pressure inducing feature which draws the sample portion through the sample probe, and these variations should be considered to fall within the scope of the present invention. For instance, the discharge apertures could be located in the side surfaces of the back portion of the sensor well, and in fluid communication with low pressure regions formed in the fluid as it speeds around the side surfaces of the sensor well. Modifications could also be made to the cross-sectional shape of the sensor well (as seen in FIG. 11) that would alter the position of the low pressure region, with a corresponding adjustment to the location of the discharge apertures to place the discharge apertures into alignment with the lower pressure regions to create the passive negative pressure inducing feature.

Although the blunt body of the sensor well 150 is configured to divert and redirect the portion of the main fluid flow 106 that is passing near to the sensor well, the sensor well is also strategically located near the wall of the fluid conduit 160 where the fluid velocity is less than the fluid velocity proximate the center of the fluid conduit. This allows the sensor well 150 to induce the low pressure/negative pressure aspects of the sensor module without simultaneously introducing a substantial obstruction into the main fluid flow that would otherwise create a detrimental increase in back pressure which could choke the engine, especially in situations where the sensor module is scaled to differently-sized fluid systems.

The blunt body of the sensor well 150 in combination with the placement of the discharge aperture 158 is a passive negative pressure inducing feature. Other passive negative pressure inducing features can be used, such as restriction devices and venturi-aspirator type arrangements. Alternatively, or additionally, the negative pressure inducing feature can be an active feature, such as a pump. In certain implementations, such as for applications having a convergent outlet pipe section downstream of a fluid treatment device, the sensor module 110 includes an extension line from the sensor well to an inlet of the outlet pipe section instead of a pressure inducing feature. Such implementations take advantage of the low pressure conditions developed at the inlet of a convergent pipe section to create the positive pressure differential.

The sensor module 110 associated with FIGS. 9-11 can be defined as a non-bypass sensor module. More specifically, because the sample probe 130 of the sensor module 110 releases the sampled portion of fluid (e.g., sample flow 118) into the approximately same space as the sample probe, the sensor modules are non-bypass sensor modules. For example, referring back to the SCR system embodiment shown in FIG. 2, both the inlet apertures and discharge aperture of the sample probe 130 are located within a space defined between two SCR catalyst beds 170, 172.

In many cases the non-bypass sensor modules 110 are able to draw sample portions 116 of the fluid flowing through the fluid conduit 160 into the sample probe 130 by virtue of a negative pressure inducing element, such as a blunt shape of the sensor well 150 (FIG. 10). However, in some instances, the use of a negative pressure inducing element may not be desirable or feasible depending on the particular application. For example, some particular types of negative pressure inducing elements may negatively alter the pattern of fluid flowing into a downstream treatment device. Accordingly, in some embodiments, a bypass sensor module can be used.

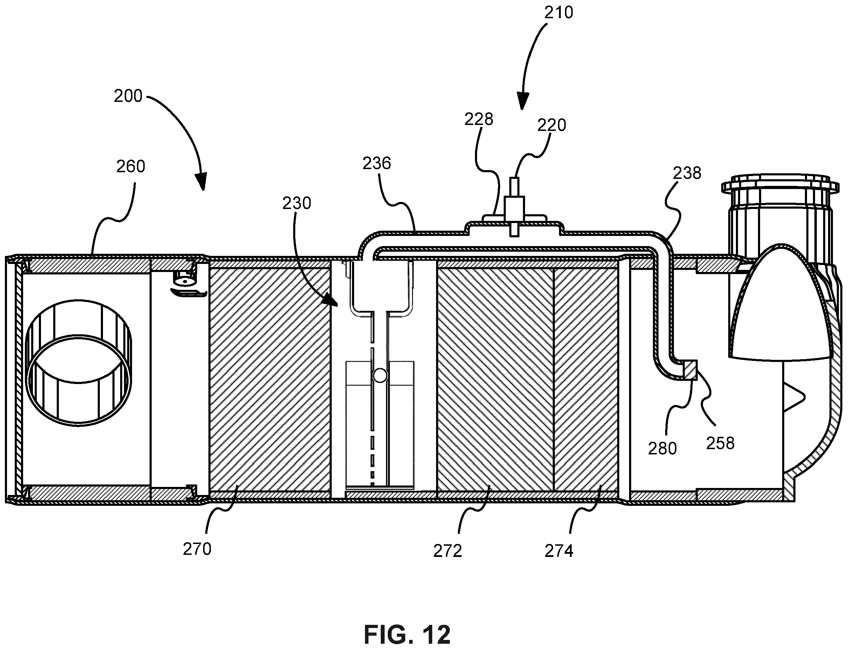

Referring to FIG. 12, a bypass sensor module 210 according to one embodiment is utilized in an SCR system 200 similar to the SCR system 100 of FIG. 2. The sensor module 210 includes a sample probe 230 having sample arms and inlet apertures configured in the same or similar manner as the sample probe described above, so as to capture a representative mix of all the constituents of the exhaust gas currently passing around and between the sample arms of the sample probe 230. However, the sample probe 230 does not provide a near immediate return of sampled fluid from the sample probe to the fluid passing through the fluid conduit. Rather, a fluid sampling line 236 extends away from the sample probe 230 to bypass a downstream fluid treatment device (e.g., the second SCR catalyst bed 272) and place the discharge aperture 258 from the sensor well at the end of a fluid outlet line 238 located downstream of the downstream fluid treatment device. Such a configuration utilizes the inherent positive pressure differential created across the downstream fluid treatment device to draw sample portions of fluid into the sample probe 230. In this manner, a passive or active negative pressure inducing feature is not required at the discharge aperture of the sensor well. Therefore, the potential for negatively affecting the fluid flow pattern that may be associated with a negative pressure inducing feature is reduced when using a bypass sensor module such as sensor module 210.

In some embodiments, the sensor module 210 includes a restriction device 280 at discharge aperture 258 to adjust (e.g., optimize) the pressure differential by restricting or blocking a portion of the exhaust flowing through the outlet line 238. The restriction device can be any of various restriction devices, such as, for example, orifice plates, perforated plates, and the like.

The fluid sampling line (i.e., bypass line) of a bypass sensor module can be configured in various ways depending on the application. As illustrated, the fluid sampling line 236 extends out of the housing 260 upstream of the downstream SCR catalyst bed 272 and runs adjacent an outer surface of the housing in an exhaust flow direction. From the fluid sampling line 236, the fluid outlet line 238 extends back into the housing downstream of the downstream SCR catalyst bed 272 and AMOX catalyst bed 274. As illustrated, the fluid outlet line 238 extends into the exhaust stream such that the discharge aperture 258 of the fluid outlet line 238 expels exhaust into a central portion of the main exhaust stream generally in the exhaust flow direction. In other embodiments, however, the fluid outlet line 238 can terminate at the housing wall such that the discharge aperture 258 of the fluid outlet line is formed in the wall to expel exhaust into a radially outward portion of the main exhaust stream. In another implementation, a small portion of the fluid outlet line 238 can extend into the main exhaust stream to form a downstream angled outlet proximate the housing wall. Other configurations for locating the discharge aperture 258 of the sample probe 230 downstream of the downstream catalyst beds 272, 274 are also possible, and are considered to fall within the scope of the present invention.

The sensor receptacle 228 and sensor 220 are located on the fluid sampling line 236 at some location between the sample probe 230 and fluid outlet line 236. Because the fluid pressure downstream of the downstream SCR catalyst bed 272 is lower than the fluid pressure upstream of the downstream SCR catalyst bed, a representative portion of the main exhaust stream is drawn into the sample probe 230, along the fluid sampling line 236, and into the fluid outlet line 238. In alternative embodiments, the fluid sampling line 236 does not extend outside of the housing 260, but extends through the downstream SCR catalyst bed 272 and AMOX catalyst bed 274.

Although the sensors of the several specific implementations for exhaust treatment applications described above have been categorized as one of ammonia and $NO_x$ sensors, in other implementations, the sensors can be other types of sensors, such as, for example, hydrocarbon sensors, carbon monoxide sensors, and the like. Alternatively, in applications outside of exhaust treatment, the sensors can be any of various sensors for sensing any of various fluid flow characteristics as desired. Furthermore, the sensors described herein can be placed at any of various locations within an exhaust aftertreatment system to sense the any of various characteristics or components (e.g., mass concentrations of constituents within the exhaust gas stream).

**Claims**

1. A sensor module (46, 110, 112) for sensing characteristics of a fluid flowing through a fluid conduit (160), comprising:

   a sample probe (130) adapted, in use, to be at least partially positioned within the fluid conduit (160) with an upstream facing side and a downstream facing side, the sample probe (130) comprising:

at least one hollow sample arm (140, 142) having a radially outer end (145) and an inner end and that, in use, extends radially inwardly from a sidewall portion of the fluid conduit (160) to a center portion of the fluid conduit (160), the at least one hollow sample arm (140, 142) including a fluid flow channel (144) formed therein and comprising a plurality of upstream facing inlet apertures (146) communicating with the fluid flow channel (144); and
a sensor well (150) located at the radially outer end (145) of the at least one sample arm (140, 142), the sensor well (150) having an interior volume (152) in fluid communication with the fluid flow channel (144), the sensor well (150) comprising a closed upstream facing front portion (157) and a downstream facing back portion (159) having a discharge aperture (158) formed therein; and

a sensor (120) positioned at least partially within the interior volume (152) of the sensor well (150): **characterized in that**:
the front portion (157) of the sensor well (150) has a blunt, rounded front surface; and **in that**:
a volume per unit length of the fluid flow channel (144) is smaller than a volume per unit length of the interior volume (152) of the sensor well (150), such that the at least one hollow sample arm (140, 142) has a width less than a width of the sensor well (150).

2. The sensor module of claim 1, wherein the plurality of inlet apertures (146) captures a sample portion (116) of fluid (106) flowing through the fluid conduit (160) and directs the sample portion (116) of fluid (106) into the interior volume (152) of the sensor well (150), the sensor (120) being in fluid communication with the sample portion (116) of fluid in the interior volume (152).

3. The sensor module (46, 110, 112) of claim 1, wherein the sensor well (150) has a substantially ovular cross-sectional shape.

4. The sensor module (46, 110, 112) of claim 1, wherein the sample probe (130) comprises a plurality of hollow sample arms (140, 142) coupled together at a central portion (143) and extending radially outwardly therefrom, each of the hollow sample arms (140, 142) defining a fluid flow channel (144), wherein the fluid flow channels (144) of the plurality of sample arms (140, 142) are fluidly coupled at the central portion (143), and wherein the sensor well (150) is located at the radially outer end (145) of one of the plurality of sample arms (140, 142).

5. The sensor module (46, 110, 112) of claim 1, wherein the plurality of inlet apertures (146) are spaced closer together near the radially outer end (145) of the at least one hollow sample arm (140, 142) than near the inner end thereof.

6. The sensor module (46, 110, 112) of claim 5, wherein for each inlet aperture (146) $j$ of the plurality of inlet apertures (146) in one hollow sample arm (140, 142), the distance of the aperture (146) away from the radially outer end (145) of the one hollow sample arm (140, 142) is equal to

$$\frac{D}{2}\left(1 - \sqrt{\frac{2n - 2j + 1}{2n}}\right)$$

where D is the diameter of a fluid conduit (160) in which the sample module (130) is intended to be used and n is the number of inlet apertures in the one hollow sample arm (140, 142).

7. The sensor module (46, 110, 112) of claim 1, further comprising a screen (124) positioned within the interior volume (152) of the sensor well (150), wherein the screen (124) envelopes the sensor (120).

8. The sensor module (46, 110, 112) of claim 1, wherein the discharge aperture (158) has a semi-circular cross-sectional shape.

9. The sensor module (46, 110, 112) of claim 1 further comprising an arcuate lower fairing (134) coupled to the radially outer end (145) of the at least one sample arm (140, 142) opposite the sensor well (150).

10. The sensor module (46, 110, 112) of claim 1 further comprising an arcuate upper fairing (132) coupled to the rounded front portion (157) and the back portion (159) of the sensor well (150).

11. An exhaust after-treatment system (100) having a sensor module (46, 110, 112) according to claim 1 installed in a fluid conduit (160) thereof.

12. The exhaust after-treatment system (100) of claim 11 further comprising an upstream SCR catalyst bed (170) positioned in the fluid conduit (160) and a downstream SCR catalyst bed (172) positioned in the fluid conduit (160), wherein the sensor module (46, 110, 112) of claim 1 is positioned between the upstream SCR catalyst bed (170) and the downstream SCR catalyst bed (172) in the fluid conduit (160).

13. A method for sensing characteristics of a fluid flowing through a fluid conduit (160) using the sensor module according to claim 1, comprising:

capturing portions (116) of the fluid (106) at a plurality of radial locations along a cross-section of the fluid conduit (160), via the inlet apertures (146);

directing the captured portions (116) of the fluid (106) through the fluid flow channel (144) of the at least one hollow sample arm (140, 142) into the sensor well (150) located at a radially outer portion of the fluid conduit (160);

passing the captured portions (116) of the fluid (160) across the sensor (120) located in the sensor well (150);

creating a low pressure region (108) in the fluid (106) flowing through the fluid conduit (160) at the radially outer portion of the fluid conduit (160) adjacent and downstream of the sensor well (150); and

drawing the captured fluid in the sensor well (150) back into the fluid conduit (160) through the discharge aperture (158) in the sensor well (150) via a pressure differential created by the low pressure region (108).

## Patentansprüche

1.  Ein Sensormodul (46, 110, 112) zum Abfühlen von Eigenschaften eines durch einen Fluidkanal (160) strömenden Fluids, beinhaltend:

    eine Prüfsonde (130), die angepasst ist, um im Einsatz mindestens teilweise in dem Fluidkanal (160) positioniert zu sein, mit einer stromaufwärts weisenden Seite und einer stromabwärts weisenden Seite, wobei die Prüfsonde (130) Folgendes beinhaltet:

    mindestens einen hohlen Prüfarm (140, 142), der ein in Radialrichtung äußeres Ende (145) und ein inneres Ende aufweist und der sich im Einsatz von einem Seitenwandabschnitt des Fluidkanals (160) zu einem Mittenabschnitt des Fluidkanals (160) in Radialrichtung nach innen erstreckt, wobei der mindestens eine hohle Prüfarm (140, 142) einen darin gebildeten Fluidströmungsdurchgang (144) umfasst und eine Vielzahl von stromaufwärts weisenden Einlassöffnungen (146) beinhaltet, die mit dem Fluidströmungsdurchgang (144) in Verbindung stehen; und

    eine an dem in Radialrichtung äußeren Ende (145) des mindestens einen Prüfarms (140, 142) befindliche Tauchhülse (150), wobei die Tauchhülse (150) ein mit dem Fluidströmungsdurchgang (144) in Verbindung stehendes Innenvolumen (152) aufweist, wobei die Tauchhülse (150) einen geschlossenen stromaufwärts weisenden vorderen Abschnitt (157) und einen stromabwärts weisenden hinteren Abschnitt (159) mit einer darin gebildeten Auslassöffnung (158) beinhaltet; und

    einen Sensor (120), der mindestens teilweise in dem Innenvolumen (152) der Tauchhülse (150) positioniert ist; **dadurch gekennzeichnet, dass**:

    der vordere Abschnitt (157) der Tauchhülse (150) eine stumpfe, abgerundete vordere Oberfläche aufweist; und dadurch, dass:

    ein Volumen pro Längeneinheit des Fluidströmungsdurchgangs (144) kleiner ist als ein Volumen pro Längeneinheit des Innenvolumens (152) der Tauchhülse (150), sodass der mindestens eine hohle Prüfarm (140, 142) eine Breite aufweist, die geringer ist als eine Breite der Tauchhülse (150).

2.  Sensormodul gemäß Anspruch 1, wobei die Vielzahl von Einlassöffnungen (146) einen Probenabschnitt (116) von durch den Fluidkanal (160) strömendem Fluid (106) erfasst und den Probenabschnitt (116) von Fluid (106) in das Innenvolumen (152) der Tauchhülse (150) leitet, wobei der Sensor (120) mit dem Probenabschnitt (116) von Fluid in dem Innenvolumen (152) in Verbindung steht.

3.  Sensormodul (46, 110, 112) gemäß Anspruch 1, wobei die Tauchhülse (150) eine im Wesentlichen ovoide Querschnittsform aufweist.

4.  Sensormodul (46, 110, 112) gemäß Anspruch 1, wobei die Prüfsonde (130) eine Vielzahl von hohlen Prüfarmen (140, 142) beinhaltet, die an einem mittleren Abschnitt (143) aneinander gekoppelt sind und sich davon in Radialrichtung nach außen erstrecken, wobei die hohlen Prüfarme (140, 142) jeweils einen Fluidströmungsdurchgang (144) definieren, wobei die Fluidströmungsdurchgänge (144) der Vielzahl von Prüfarmen (140, 142) an dem mittleren Abschnitt (143) fluidisch gekoppelt sind, und wobei sich die Tauchhülse (150) an dem in Radialrichtung äußeren Ende (145) eines der Vielzahl von Prüfarmen (140, 142) befindet.

5.  Sensormodul (46, 110, 112) gemäß Anspruch 1, wobei die Vielzahl von Einlassöffnungen (146) in der Nähe des in Radialrichtung äußeren Endes (145) des mindestens einen hohlen Prüfarms (140, 142) in engeren Abständen angeordnet sind als in der Nähe des inneren Endes desselben.

6.  Sensormodul (46, 110, 112) gemäß Anspruch 5, wobei für jede Einlassöffnung (146) *j* der Vielzahl von Einlassöffnungen (146) in einem hohlen Prüfarm (140, 142) der Abstand der Öffnung (146) von dem in Radialrichtung äußeren Ende (145) des einen hohlen Prüfarms (140, 142) gleich

$$\frac{D}{2}\left(1 - \sqrt{\frac{2n - 2j + 1}{2n}}\right)$$

ist, wobei D der Durchmesser eines Fluidkanals (160) ist, in dem die Verwendung des Prüfmoduls (130) beabsichtigt ist, und n die Zahl der Einlassöffnungen in dem einen hohlen Prüfarm (140, 142) ist.

7. Sensormodul (46, 110, 112) gemäß Anspruch 1, ferner beinhaltend ein in dem Innenvolumen (152) der Tauchhülse (150) positioniertes Sieb (124), wobei das Sieb (124) den Sensor (120) umhüllt.

8. Sensormodul (46, 110, 112) gemäß Anspruch 1, wobei die Auslassöffnung (158) eine halbkreisförmige Querschnittsform aufweist.

9. Sensormodul (46, 110, 112) gemäß Anspruch 1, ferner beinhaltend eine bogenförmige untere Verkleidung (134), die gegenüber der Tauchhülse (150) an das in Radialrichtung äußere Ende (145) des mindestens einen Prüfarms (140, 142) gekoppelt ist.

10. Sensormodul (46, 110, 112) gemäß Anspruch 1, ferner beinhaltend eine bogenförmige obere Verkleidung (132), die an den abgerundeten vorderen Abschnitt (157) und den hinteren Abschnitt (159) der Tauchhülse (150) gekoppelt ist.

11. Ein Abgasnachbehandlungssystem (100), das ein in einem Fluidkanal (160) desselben installiertes Sensormodul (46, 110,112) gemäß Anspruch 1 aufweist.

12. Abgasnachbehandlungssystem (100) gemäß Anspruch 11, ferner beinhaltend ein in dem Fluidkanal (160) positioniertes stromaufwärtiges SCR-Katalysatorbett (170) und ein in dem Fluidkanal (160) positioniertes stromabwärtiges SCR-Katalysatorbett (172), wobei das Sensormodul (46, 110, 112) gemäß Anspruch 1 zwischen dem stromaufwärtigen SCR-Katalysatorbett (170) und dem stromabwärtigen SCR-Katalysatorbett (172) in dem Fluidkanal (160) positioniert ist.

13. Ein Verfahren zum Abfühlen von Eigenschaften eines durch einen Fluidkanal (160) strömenden Fluids unter Verwendung des Sensormoduls gemäß Anspruch 1, beinhaltend:

Erfassen von Abschnitten (116) des Fluids (106) an einer Vielzahl von Stellen in Radialrichtung entlang eines Querschnitts des Fluidkanals (160) mittels der Einlassöffnungen (146);
Führen der erfassten Abschnitte (116) des Fluids (106) durch den Fluidströmungsdurchgang (144) des mindestens einen hohlen Prüfarms (140, 142) in die an einem in Radialrichtung äußeren Abschnitt des Fluidkanals (160) befindliche Tauchhülse (150);
Leiten der erfassten Abschnitte (116) des Fluids (160) über den in der Tauchhülse (150) befindlichen Sensor (120);
Erzeugen eines Niederdruckbereichs (108) in dem durch den Fluidkanal (160) strömenden Fluid (106) an dem in Radialrichtung äußeren Abschnitt des Fluidkanals (160), der Tauchhülse (150) benachbart und stromabwärts derselben; und
Saugen des erfassten Fluids in der Tauchhülse (150) zurück in den Fluidkanal (160) durch die Auslassöffnung (158) in der Tauchhülse (150) mittels eines von dem Niederdruckbereich (108) erzeugten Druckdifferentials.

## Revendications

1. Un module détecteur (46, 110, 112) pour détecter des caractéristiques d'un fluide s'écoulant à travers un conduit de fluide (160), comprenant :

une sonde d'échantillon (130) conçue, lors de l'utilisation, pour être au moins partiellement positionnée à l'intérieur du conduit de fluide (160) avec un côté tourné vers l'amont et un côté tourné vers l'aval, la sonde d'échantillon (130) comprenant :

au moins un bras d'échantillon creux (140, 142) ayant une extrémité radialement externe (145) et une extrémité interne et qui, lors de l'utilisation, s'étend radialement vers l'intérieur à partir d'une portion de paroi latérale du conduit de fluide (160) jusqu'à une portion centrale du conduit de fluide (160), l'au moins un bras d'échantillon creux (140, 142) incluant un canal d'écoulement de fluide (144) formé à l'intérieur de celui-ci et comprenant une pluralité d'ouvertures d'entrée tournées vers l'amont (146) communiquant avec le canal d'écoulement de fluide (144) ; et
un puits de détecteur (150) situé au niveau de l'extrémité radialement externe (145) de l'au moins un bras d'échantillon (140, 142), le puits de détecteur (150) ayant un volume intérieur (152) en communication fluidique avec le canal d'écoulement de fluide (144), le puits de détecteur (150) comprenant une portion avant tournée vers l'amont fermée (157) et une portion arrière tournée vers l'aval (159) ayant une ouverture d'évacuation (158) formée à l'intérieur de celle-ci ; et

un détecteur (120) positionné au moins partiellement à l'intérieur du volume intérieur (152) du puits de détecteur (150) : **caractérisé en ce que** :
la portion avant (157) du puits de détecteur (150) a une surface avant arrondie émoussée ; et **en ce que** :
un volume par unité de longueur du canal d'écoulement de fluide (144) est plus petit qu'un volume par unité de longueur du volume intérieur (152) du puits de détecteur (150), de telle sorte que l'au moins un bras d'échantillon creux (140, 142) ait une largeur moindre qu'une largeur du puits de détecteur (150).

2. Le module détecteur de la revendication 1, où la pluralité d'ouvertures d'entrée (146) capture une portion échantillon (116) de fluide (106) s'écoulant à travers le conduit de fluide (160) et oriente la portion échantillon (116) de fluide (106) jusque dans le volume intérieur (152) du puits de détecteur (150), le détecteur (120) étant en communication fluidique avec la portion échantillon (116) de fluide dans le volume intérieur (152).

3. Le module détecteur (46, 110, 112) de la revendication 1, où le puits de détecteur (150) a une forme en coupe transversale substantiellement ovulaire.

4. Le module détecteur (46, 110, 112) de la revendication 1, où la sonde d'échantillon (130) comprend une pluralité de bras d'échantillon creux (140, 142) couplés les uns aux autres au niveau d'une portion centrale (143) et s'étendant radialement vers l'extérieur à partir de celle-ci, chacun des bras d'échantillon creux (140, 142) définissant un canal d'écoulement de fluide (144), les canaux d'écoulement de fluide (144) de la pluralité de bras d'échantillon (140, 142) étant couplés de manière fluidique au niveau de la portion centrale (143), et où le puits de détecteur (150) est situé au niveau de l'extrémité radialement extérieure (145) d'un bras de la pluralité de bras d'échantillon (140, 142).

5. Le module détecteur (46, 110, 112) de la revendication 1, où la pluralité d'ouvertures d'entrée (146) sont espacées les unes des autres de manière plus rapprochée près de l'extrémité radialement externe (145) de l'au moins un bras d'échantillon creux (140, 142) que près de l'extrémité interne de celui-ci.

6. Le module détecteur (46, 110, 112) de la revendication 5, où pour chaque ouverture d'entrée (146) *j* de la pluralité d'ouvertures d'entrée (146) dans un bras d'échantillon creux (140, 142), la distance de l'ouverture (146) à distance de l'extrémité radialement externe (145) de ce bras d'échantillon creux (140, 142) est égale à

$$\frac{D}{2}\left(1 - \sqrt{\frac{2n - 2j + 1}{2n}}\right)$$

où D est le diamètre d'un conduit de fluide (160) dans lequel le module échantillon (130) est destiné à être utilisé et *n* est le nombre d'ouvertures d'entrée dans ce bras d'échantillon creux (140, 142).

7. Le module détecteur (46, 110, 112) de la revendication 1, comprenant en outre un écran (124) positionné à l'intérieur du volume intérieur (152) du puits de détecteur (150), l'écran (124) enveloppant le détecteur (120).

8. Le module détecteur (46, 110, 112) de la revendication 1, où l'ouverture d'évacuation (158) a une forme en coupe transversale semi-circulaire.

9. Le module détecteur (46, 110, 112) de la revendication 1 comprenant en outre un carénage inférieur arqué (134) couplé à l'extrémité radialement externe (145) de l'au moins un bras d'échantillon (140, 142) à l'opposé du puits de détecteur (150).

10. Le module détecteur (46, 110, 112) de la revendication 1 comprenant en outre un carénage supérieur arqué (132) couplé à la portion avant arrondie (157) et la portion arrière (159) du puits de détecteur (150).

11. Un système post-traitement d'échappement (100) ayant un module détecteur (46, 110, 112) selon la revendication 1 installé dans un conduit de fluide (160) de celui-ci.

12. Le système post-traitement d'échappement (100) de la revendication 11 comprenant en outre un lit catalytique SCR amont (170) positionné dans le conduit de fluide (160) et un lit catalytique SCR aval (172) positionné dans le conduit de fluide (160), où le module détecteur (46, 110, 112) de la revendication 1 est positionné entre le lit catalytique SCR amont (170) et le lit catalytique SCR aval (172) dans le conduit de fluide (160).

13. Un procédé pour détecter des caractéristiques d'un fluide s'écoulant à travers un conduit de fluide (160) à l'aide du module détecteur selon la revendication 1, comprenant :

la capture de portions (116) du fluide (106) au niveau d'une pluralité d'emplacements radiaux le long d'une coupe transversale du conduit de fluide (160), par l'intermédiaire des ouvertures d'entrée (146) ;

l'orientation des portions capturées (116) du fluide (106) à travers le canal d'écoulement de fluide (144) de l'au moins un bras d'échantillon creux (140, 142) jusque dans le puits de détecteur (150) situé au niveau d'une portion radialement externe du conduit de fluide (160) ;

le passage des portions capturées (116) du fluide (160) d'un bout à l'autre du détecteur (120) situé dans le puits de détecteur (150) ;

la création d'une région basse pression (108) dans le fluide (106) s'écoulant à travers le conduit de fluide (160) au niveau de la portion radialement externe du conduit de fluide (160) adjacente et en aval du puits de détecteur (150) ; et

l'aspiration du fluide capturé dans le puits de détecteur (150) en retour jusque dans le conduit de fluide (160) à travers l'ouverture d'évacuation (158) dans le puits de détecteur (150) par l'intermédiaire d'un différentiel de pression créé par la région basse pression (108).

**FIG. 1**

**FIG. 2**

**FIG. 3**

**FIG. 4**

**FIG. 5**

**FIG. 6**

FIG. 8

FIG. 7

FIG. 9

**FIG. 10**

**FIG. 11**

**FIG. 12**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10203310 A1